# EUROPEAN PATENT SPECIFICATION

(11) **EP 4 313 278 B1**
(45) Date of publication and mention of the grant of the patent: **01.04.2026**
(21) Application number: 22740646.9
(22) Date of filing: 06.06.2022
(51) Int. Cl.: A61N 1/372, G16H 40/40

(54) **STIMULATION SYSTEMS WITH USER-SPECIFIED ROUTINES**
STIMULATIONSSYSTEME MIT BENUTZERSPEZIFIZIERTENROUTINEN
SYSTÈMES DE STIMULATION AYANT DES ROUTINES SPÉCIFIÉES PAR L'UTILISATEUR

(30) Priority: 07.06.2021 US 202163197747 P
(43) Date of publication of application: 07.02.2024
(73) Proprietor: Boston Scientific Neuromodulation Corporation, Valencia, CA 91355 (US)
(72) Inventor: HUYNH, Dat, Thanh, Hollywood, CA 90046 (US); MCDONALD, Matthew, Lee, Glendale, CA 91208 (US)
(74) Representative: Vossius & Partner Patentanwälte Rechtsanwälte mbB
(86) International application number: PCT/US2022/032349
(87) International publication number: WO 2022/261001

(56) References cited:
- WO-A1-2016/112398
- US-A1- 2007 260 283
- US-A1- 2017 197 086
- US-B1- 6 516 227

## Description

### CROSS-REFERENCE TO RELATED APPLICATIONS

This application claims the benefit under 35 U.S.C. § 119(e) of U.S. Provisional Patent Application Serial No. 63/197,747, filed June 7, 2021.

### FIELD

The present disclosure is directed to the area of stimulation systems including user-specified routines for stimulation and methods of making and using. The present disclosure is also directed to methods and systems including stimulation systems including user-specified routines for stimulation activated in response to external information and methods of making and using.

### BACKGROUND

Implantable electrical stimulation systems have proven therapeutic in a variety of diseases and disorders. For example, deep brain stimulation systems have been used as a therapeutic modality for the treatment of Parkinson's disease, essential tremor, and the like.

Stimulators have been developed to provide therapy for a variety of treatments. A stimulator can include an implantable pulse generator (IPG), one or more leads, and an array of stimulator electrodes on each lead. The stimulator electrodes are in contact with or near the nerves, muscles, or other tissue to be stimulated. The pulse generator in the IPG generates electrical pulses that are delivered by the electrodes to body tissue.

Implantable medical devices (IMDs), including IPGs, typically have the capability to communicate data with an external device, such as a clinician programmer or a remote control, via a radio-frequency telemetry link or other wireless communication method. The clinician programmer can program the operating parameters of the implanted medical device. The remote control can switch programs. Modern implantable devices also include the capability for bidirectional communication so that information can be transmitted to the clinician programmer or remote control from the implanted device.

U.S. Patent Application Publication No. 2017/0197086 describes a system that may include a processor subsystem; and a memory device comprising instructions, which when executed by the processor subsystem, cause the processor subsystem to: receive at an application executing on a user device, a request to modify the application; transmit the request to an administrative user device for approval; receive a response to the request from the administrative user device; and modify a functionality of the application in response to receiving the response.

### BRIEF SUMMARY

One aspect is a stimulation system that includes a device programmed with a plurality of user-specified routines for operation of a pulse generator, wherein the user-specified routines are activatable in response to information received from a source external to the pulse generator, The device includes a memory upon which the user-specified routines are stored, a communication unit configured to receive or send signals, and at least one processor coupled to the memory and the communication unit and configured to perform actions, including receiving, using the communication unit, information provided from the source external to the pulse generator and external to the patient, when the information meets an activation criterion for a one of the user-specified routines, activating the user-specified routine to deliver or halt delivery of stimulation to the patient according to the user-specified routine, and upon completion of the user-specified routine or meeting a termination condition, halting the user-specified routine.

In at least some aspects, the device is the pulse generator which is an implantable pulse generator or an external trial stimulator. In at least some aspects, the stimulation system further includes a sensor including an antenna for communicating with the device and providing the information. In at least some aspects, the information is a sensor measurement from the sensor. In at least some aspects, the sensor is configured to provide a sensor measurement indicating at least one of the following: movement by the patient, that the patient is asleep, that the patient is driving, or that the patient is exercising. In at least some aspects, the sensor is configured to provide a sensor measurement indicating at least one of the following: pain, disease/disorder state, or disease/disorder progression.

In at least some aspects, the user-specified routines are activatable in response to information received from the source that is external to the patient. In at least some aspects, the user-specified routines are activatable in response to information received from the source that is external to the device.

In at least some aspects, the stimulation system further includes a remote control, clinician programmer, or mobile device including a communication unit for communicating with the device and providing the information. In at least some aspects, the information is a location of the patient or weather information.

In at least some aspects, the stimulation system further includes a charger, wherein the source of the information is the charger. In at least some aspects, the device is a remote control, clinician programmer, smartphone, or smartwatch configured for communicating with the pulse generator using the communication unit, wherein the actions further include, when the information meets the activation criterion for the one of the user-specified routines and prior to activating the one of the user-specified routines, presenting a message on the remote control, clinician programmer, smartphone, or smartwatch requesting confirmation of the activation of the one of the user-specified routines by the patient.

Another aspect is a stimulation system that includes a display; at least one user input device; and at least one processor coupled to the display and user input device, the at least one processor configured to perform actions, including: providing a user interface on the display; receiving, via the at least one user input device, a user-selection of a stimulation program for a user-specified routine for stimulation of a patient using a pulse generator; receiving, via the at least one user input device, a user-selection of an activation criterion for the user-specified routine; and receiving, via the at least one user input device, a user-selection of a source external to the pulse generator to provide the activation criterion.

In at least some aspects, the actions further include initiating a signal to program the pulse generator to include the user-selection of the stimulation program, activation criterion, and source for the user-specified routine. In at least some aspects, the actions further including receiving, via the at least one user input device, a termination. In at least some aspects, receiving the user-selection of the activation criterion and receiving the user-selection of the source include receiving, via the at least one user input device, a user-selection of a predefined routine from a database.

In at least some aspects, the actions further include presenting a suggested routine to the user on the user interface based on observations of previous patient behavior or previous user or patient selections of stimulation programs. In at least some aspects, the actions further include receiving, via the at least one user input device, a user-selection of an option to require confirmation of an activation of the user-specified routine by the user when activating the user-specified routine.

In at least some aspects, the stimulation system further includes a remote control, clinician programmer, or mobile device, wherein the display, the user input device, and the at least one processor are part of the remote control, clinician programmer, smartphone, smartwatch, tablet, or computer. In at least some aspects, the stimulation system further includes the pulse generator

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic view of one embodiment of an electrical stimulation system that includes one or more leads that can be coupled to an IPG;
FIG. 2 is a block diagram of element of an electrical stimulation system;
FIG. 3 is a flowchart of one embodiment of a method of programming a routine;
   and
FIG. 4 is a flowchart of one embodiment of a method of performing a routine using a pulse generator.

### DETAILED DESCRIPTION

The present disclosure is directed to the area of stimulation systems including user-specified routines for stimulation and methods of making and using. The present disclosure is also directed to methods and systems including stimulation systems including user-specified routines for stimulation activated in response to external information and methods of making and using.

Implantable electrical stimulation systems and devices are used herein to exemplify the inventions, but it will be understood that these inventions can be utilized with other stimulation systems and devices. Examples of implantable electrical stimulation systems include, but are not limited to, a least one lead with one or more electrodes disposed along a distal end of the lead and one or more terminals disposed along the one or more proximal ends of the lead. Examples of electrical stimulation systems with leads are found in, for example, U.S. Patents Nos. 6,181,969; 6,295,944; 6,391,985; 6,516,227; 6,609,029; 6,609,032; 6,741,892; 7,244,150; 7,450,997; 7,672,734;7,761,165; 7,783,359; 7,792,590; 7,809,446; 7,949,395; 7,974,706; 8,831,742; 8,688,235; 8,175,710; 8,224,450; 8,271,094; 8,295,944; 8,364,278; and 8,391,985; U.S. Patent Application Publications Nos. 2007/0150036; 2009/0187222; 2009/0276021; 2010/0076535; 2010/0268298; 2011/0004267; 2011/0078900; 2011/0130817; 2011/0130818; 2011/0238129; 2011/0313500; 2012/0016378; 2012/0046710; 2012/0071949; 2012/0165911; 2012/0197375; 2012/0203316; 2012/0203320; 2012/0203321; 2012/0316615; 2013/0105071; 2011/0005069; 2010/0268298; 2011/0130817; 2011/0130818; 2011/0078900; 2011/0238129; 2011/0313500; 2012/0016378; 2012/0046710; 2012/0165911; 2012/0197375; 2012/0203316; 2012/0203320; and 2012/0203321.

In addition, the methods, techniques, and systems described herein are presented in the context of an electrical stimulation system, but it will be recognized that these methods, techniques, and systems can be used with an optical stimulation system or an electrical/optical stimulation system. Examples of optical stimulation systems or electrical/optical stimulation systems are found in U.S. Patent Application Publications Nos. 2013/0317572; 2013/0317573; 2017/0259078; 2017/0225007; 2018/0110971; 2018/0369606; 2018/0369607; 2019/0209849; 2019/0209834; 2020/0094047; and 2020/0155854 and U.S. Patent Application No. 16/883,404.

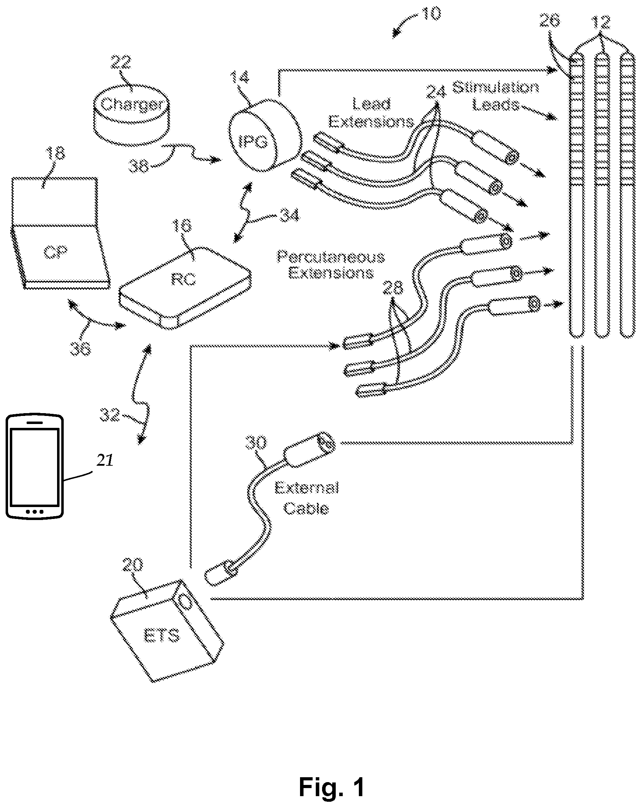

Turning to Figure 1, one embodiment of an electrical stimulation system 10 includes one or more stimulation leads 12 and an implantable pulse generator (IPG) 14. The system 10 can also include one or more of an external remote control (RC) 16, a clinician's programmer (CP) 18, an external trial stimulator (ETS) 20, an external charger 22, or another device 21(such as a smartphone, tablet, smartwatch, personal data assistant, laptop computer, desktop computer, or the like). The IPG and ETS are examples of control modules for the electrical stimulation system.

The IPG 14 is physically connected, optionally via one or more lead extensions 24, to the stimulation lead(s) 12. Each lead carries multiple electrodes 26 arranged in an array. The IPG 14 includes pulse generation circuitry that delivers electrical stimulation energy in the form of, for example, a pulsed electrical waveform (i.e., a temporal series of electrical pulses) to the electrode array 26 in accordance with a set of stimulation parameters. The implantable pulse generator can be implanted into a patient's body, for example, below the patient's clavicle area or within the patient's abdominal cavity or at any other suitable site. The implantable pulse generator 14 can have multiple stimulation channels which may be independently programmable to control the magnitude of the current stimulus from each channel. In at least some embodiments, the implantable pulse generator 14 can have any suitable number of stimulation channels including, but not limited to, 4, 6, 8, 12, 16, 32, or more stimulation channels. The implantable pulse generator 14 can have one, two, three, four, or more connector ports, for receiving the terminals of the leads and/or lead extensions.

The ETS 20 may also be physically connected, optionally via the percutaneous lead extensions 28 and external cable 30, to the stimulation leads 12. The ETS 20, which may have similar pulse generation circuitry as the IPG 14, also delivers electrical stimulation energy in the form of, for example, a pulsed electrical waveform to the electrode array 26 in accordance with a set of stimulation parameters. One difference between the ETS 20 and the IPG 14 is that the ETS 20 is often a non-implantable device that is used on a trial basis after the neurostimulation leads 12 have been implanted and prior to implantation of the IPG 14, to test the responsiveness of the stimulation that is to be provided. Any functions described herein with respect to the IPG 14 can likewise be performed with respect to the ETS 20.

The RC 16 may be used to telemetrically communicate with or control the IPG 14 or ETS 20 via a uni- or bi-directional wireless communications link 32. Once the IPG 14 and neurostimulation leads 12 are implanted, the RC 16 may be used to telemetrically communicate with or control the IPG 14 via a uni- or bi-directional communications link 34. Such communication or control allows the IPG 14, for example, to be turned on or off and to be programmed with different stimulation parameter sets. The IPG 14 may also be operated to modify the programmed stimulation parameters to actively control the characteristics of the electrical stimulation energy output by the IPG 14. In at least some embodiments, the CP 18 (or RC 16 or other programming device) allows a user, such as a clinician, the ability to program stimulation parameters for the IPG 14 and ETS 20 in the operating room and in follow-up sessions. Alternately, or additionally, in at least some embodiments, stimulation parameters can be programmed via wireless communications (e.g., Bluetooth) between the RC 16 (or other external device 21 such as a hand-held electronic device like a smartphone, tablet, or the like) and the IPG 14.

The CP 18 may perform this function by indirectly communicating with the IPG 14 or ETS 20, through the RC 16, via a wireless communications link 36. Alternatively, the CP 18 may directly communicate with the IPG 14 or ETS 20 via a wireless communications link (not shown). In at least some embodiments, the stimulation parameters provided by the CP 18 are also used to program the RC 16, so that the stimulation parameters can be subsequently modified by operation of the RC 16 in a stand-alone mode (i.e., without the assistance of the CP 18). The CP 18 or RC 16 can be any suitable device including, but not limited to, a computer or other computing device, laptop, mobile device (for example, a smartphone or tablet), or the like or any combination thereof. The CP 18, RC 16, or other device 21 can include software applications for interacting with the IPG 14 and for programming the IPG 14. The device 21 can communicate with one or more of the CP 18, RC 16, ETS 20, or IPG 14. The external charger can use a wireless link 38 to charge the IPG.

Additional examples of the RC 16, CP 18, ETS 20, and external charger 22 can be found in the references cited herein as well as U.S. Patents Nos. 6,895,280; 6,181,969; 6,516,227; 6,609,029; 6,609,032; 6,741,892; 7,949,395; 7,244,150; 7,672,734; and 7,761,165; 7,974,706; 8,175,710; 8,224,450; and 8,364,278; and U.S. Patent Application Publication No. 2007/0150036.

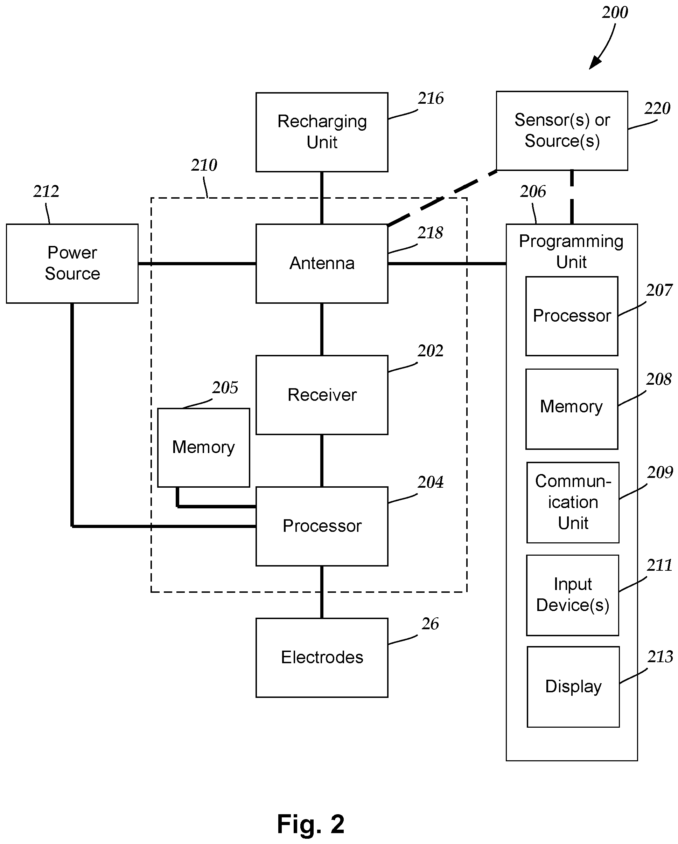

Figure 2 is a schematic overview of one embodiment of components of a stimulation system 200 including an electronic subassembly 210 disposed within an IPG 14 (Figure 1). It will be understood that the electrical stimulation system can include more, fewer, or different components and can have a variety of different configurations including those configurations disclosed in the stimulator references cited herein.

The IPG 14 (Figure 1) can include, for example, a power source 212, antenna 218 (or other communication unit), receiver 202, processor 204, and memory 205. A programming unit 206, such as a CP 18, RC 16, or other device 21 (Figure 1), can include a processor 207 and memory 208. Some of the components (for example, power source 212, antenna 218, receiver 202, processor 204, and memory 205) of the IPG 14 can be positioned on one or more circuit boards or similar carriers within a sealed housing of the IPG 14 (Figure 1), if desired. Any power source 212 can be used including, for example, a battery such as a primary battery or a rechargeable battery. Examples of other power sources include super capacitors, nuclear or atomic batteries, mechanical resonators, infrared collectors, thermally-powered energy sources, flexural powered energy sources, bioenergy power sources, fuel cells, bioelectric cells, osmotic pressure pumps, and the like including the power sources described in U.S. Patent No. 7,437,193.

As another alternative, power can be supplied by an external power source through inductive coupling via the optional antenna 218 or a secondary antenna. The external power source can be in a device that is mounted on the skin of the patient or in a unit that is provided near the patient on a permanent or periodic basis.

If the power source 212 is a rechargeable battery, the battery may be recharged using the optional antenna 218, if desired. Power can be provided to the battery for recharging by inductively coupling the battery through the antenna to a recharging unit 216 (such as charger 22 (Figure 1)) external to the patient. Examples of such arrangements can be found in the references identified above.

In one embodiment, electrical current is emitted by the electrodes 26 on the lead body to stimulate nerve fibers, muscle fibers, or other body tissues near the electrical stimulation system. A processor 204 is generally included to control the timing and electrical characteristics of the electrical stimulation system. For example, the processor 204 can, if desired, control one or more of the timing, frequency, amplitude, width, and waveform of the pulses. In addition, the processor 204 can select which electrodes can be used to provide stimulation, if desired. In some embodiments, the processor 204 may select which electrode(s) are cathodes and which electrode(s) are anodes. In some embodiments, the processor 204 may be used to identify which electrodes provide the most useful stimulation of the desired tissue. Instructions for the processor 204 can be stored on the memory 205. Instructions for the processor 207 can be stored on the memory 208.

Any processor 204 can be used for the IPG and can be as simple as an electronic device that, for example, produces pulses at a regular interval or the processor can be capable of receiving and interpreting instructions from the programming unit 206 that, for example, allows modification of pulse characteristics. In the illustrated embodiment, the processor 204 is coupled to a receiver 202 which, in turn, is coupled to the optional antenna 218. This allows the processor 204 to receive instructions from an external source to, for example, direct the pulse characteristics and the selection of electrodes, if desired. Any suitable processor 207 can be used for the programming unit 206.

Any suitable memory 205, 208 can be used including computer-readable storage media may include, but is not limited to, volatile, nonvolatile, non-transitory, removable, and non-removable media implemented in any method or technology for storage of information, such as computer readable instructions, data structures, program modules, or other data. Examples of computer-readable storage media include, but are not limited to, RAM, ROM, EEPROM, flash memory, or other memory technology, CD-ROM, digital versatile disks ("DVD") or other optical storage, magnetic cassettes, magnetic tape, magnetic disk storage or other magnetic storage devices, or any other medium which can be used to store the desired information and which can be accessed by a processor.

In one embodiment, the antenna 218 (or other communication unit) is capable of receiving signals (e.g., RF signals) from a communication unit 209 (for example, an antenna) of a programming unit 206 which is programmed or otherwise operated by a user. The signals sent to the processor 204 via the antenna 218 and receiver 202 can be used to modify or otherwise direct the operation of the electrical stimulation system. For example, the signals may be used to modify the pulses of the electrical stimulation system such as modifying one or more of pulse width, pulse frequency, pulse waveform, and pulse amplitude. The signals may also direct the electrical stimulation system 200 to cease operation, to start operation, to start charging the battery, or to stop charging the battery. In other embodiments, the stimulation system does not include an antenna 218 or receiver 202 and the processor 204 operates as programmed.

Optionally, the electrical stimulation system 200 may include a transmitter (not shown) coupled to the processor 204 and the antenna 218 for transmitting signals back to the programming unit 206 or another unit capable of receiving the signals. For example, the electrical stimulation system 200 may transmit signals indicating whether the electrical stimulation system 200 is operating properly or not or indicating when the battery needs to be charged or the level of charge remaining in the battery. The processor 204 may also be capable of transmitting information about the pulse characteristics so that a user or clinician can determine or verify the characteristics.

Transmission of signals via the antenna 218 or communication unit 209 can occur using any suitable method, technique, or platform including, but not limited to, inductive transmission, radiofrequency transmission, Bluetooth^{™}, Wi-Fi, cellular transmission, near field transmission, infrared transmission, or the like or any combination thereof. In addition, the IPG 14 can be wirelessly coupled to the RC 16 or CP 18 using any suitable arrangement include direct transmission or transmission through a network, such as a local area network, wide area network, the Internet, or the like or any combination thereof. The CP 18 or RC 16 may also be capable of coupling to, and sending data or other information to, a network, such as a local area network, wide area network, the Internet, or the like or any combination thereof.

As described in more detail below, the stimulation system 200 can also include, or have access to, one or more sensor(s) or information source(s) 220 that communicate to one or both of the IPG 14 (through antenna 218) or programming unit 206.

The programming unit 206 can include a display 213 and one or more input devices 211 to produce a user interface which can be shown on the display and receives interaction through the input device(s). Example of input devices 211 include, but are not limited to, a keyboard, mouse, track ball, touchscreen, voice recognition software, or the like or any combination thereof.

Returning to Figure 1, the CP 18 or RC 16 (or other external device) utilizes software applications to communicate with the IPG 14. These software applications are often custom-made and instructions for the software applications can be stored in the memory of the CP, RC, or other external device. In at least some embodiments, depending on its purpose, each software application may have a capability (or permission) to perform one or more of the following: read data, write data, select from existing programs, modify programming settings of existing programs, create programs, modify or update software/firmware, full control (which could include all of the previously listed capabilities), or otherwise interact with the IPG or any combination of these capabilities (or permissions).

Patients may have a routine they habitually follow when using an electrical stimulation device. For example, the patient may wish to utilize a particular electrical stimulation program after arriving home from work or when performing chores or when lying down. Using a conventional electrical stimulation system, the patient will need to find and activate their remote control and then navigate the menus to change the electrical stimulation program or adjust stimulation settings.

Scheduling electrical stimulation at particular times can be useful, but not all activities are performed on a reliable schedule. Other examples of patient preference regarding electrical stimulation therapy can include: receiving or not receiving electrical stimulation when lying in bed; receiving or not receiving electrical stimulation when performing tasks (for example, receiving spinal cord stimulation when standing and washing dishes or performing other tasks while standing); receiving or not receiving electrical stimulation depending on the weather (for example, receiving stimulation when the weather is cold); cycling through different therapy programs and picking one for current therapy; or the like or any combination thereof.

As described herein, an electrical stimulation system can include user-specified routines that select an electrical stimulation program, halt or pause electrical stimulation, or modify electrical stimulation parameters based on information (e.g., event triggers or sensor measurements) from sensors or information obtained from a source (for example, a website, a RC 16, CP 18, or any other suitable device 21) that is external to the IPG 14 or other pulse generator, such as ETS 20. In at least some embodiments, the information is obtained from a source external to the patient. In at least some embodiments, the information is obtained from a source external to the pulse generator (for example, IPG 14 or ETS 20) and a RC 16, CP 18, or any other suitable device 21 in communication with the pulse generator.

Examples of information that can be received include sensor information (e.g., sensor measurements) that indicate motion, movement, location, sleep, exercise, pain, disease/disorder state, disease/disorder progression, other states of the patient, heart rate, respiration rate, blood oxygen level, blood glucose level, pulse, posture, electroencephalogram, skin temperature, core temperature, or the like or any combination thereof. Other examples of information include patient location (for example, at home, at the gym, at the office, in a car, within a geofenced location, within a specific WiFi network, or the like or any combination thereof), weather information (for example, current weather, temperature, pressure, humidity, precipitation), attachment to a charger, questionnaire responses, voice responses, or the like or any combination thereof.

The routines may be selected or programmed by a user, such as a patient, a clinician, a programmer, or any other suitable individual or system. In at least some embodiments, the routines are stored on the IPG 14, ETS 20, or other pulse generator. In at least some embodiments, the routines are stored on on the RC 16, CP 18, or any other suitable device 21. In at least some embodiments, the routines can be triggered or activated by the IPG 14, ETS 20, or other pulse generator. In at least some embodiments, the routines can be triggered or activated by the RC 16, CP 18, or any other suitable device 21 which instructs the IPG 14, ETS 20, or other pulse generator to implement the routine. In at least some embodiments, the routines can be triggered or activated by the RC 16, CP 18, or any other suitable device 21 which then controls operation of the IPG 14, ETS 20, or other pulse generator to implement the routine.

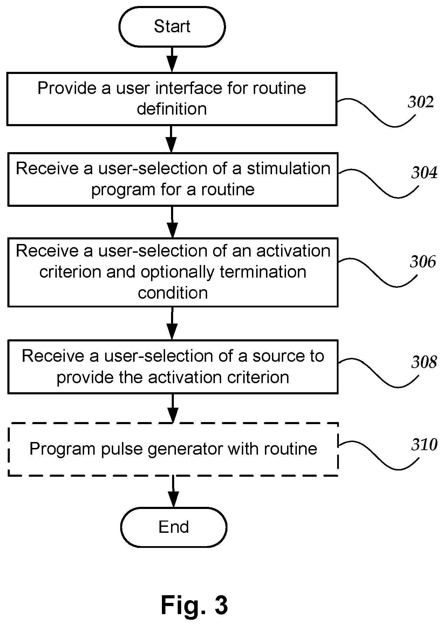

The electrical stimulation system can include a user interface to allow a user to define or program the routines. For example, the user interface may include the display 213 and input device(s) 211 of the programming unit 206 (or other suitable device) illustrated in Figure 2. Figure 3 illustrates one embodiment of a method of programming a routine. In step 302, a user interface is provided for routine definition. The user interface can be provided on (referring back to Figure 1) a RC 16, CP 18, or any other suitable device 21, such as a smartphone, smartwatch, tablet, laptop computer, desktop computer, personal data assistant, or the like.

The routine is defined or otherwise specified using the user interface. One example of a method to define or otherwise specify the routine is provided in steps 304 to 308. It will be recognized that these steps can be performed in any order. It will also be recognized that other types of information (external to the pulse generator, such as IPG 14) can be used to specify the routine. The routine is defined or otherwise specified by a user which can be a clinician, a patient, a manufacturer or representative of the manufacturer, a third party, or any combination thereof. In at least some embodiments, previously defined routines may be obtained from any suitable source and can be used or modified by a user. For example, routines may be presented in, or available from, a menu on the user interface of the RC 16, CP 18, or any other suitable device 21. In at least some embodiments, defined routines can be shared between users or obtained from a database. Such shared routines may be modifiable. In at least some embodiments, the RC 16, CP 18, or any other suitable device 21 may suggest a routine to the user on the user interface based on observations of patient behavior or previous user or patient selections of stimulation programs.

In step 304, the user selects or defines a stimulation program to be used for the routine. The stimulation program can define one or more stimulation parameters such as, but not limited to, selection of electrode(s), amplitude, pulse width, pulse frequency, duration of stimulation, or the like or any combination thereof. In at least some embodiments, the user may select from previously defined stimulation programs. In at least some embodiments, the user may alternatively or additionally select stimulation parameters to define a new stimulation program. In at least some embodiments, the stimulation program can be a program that halts or pauses stimulation or prevents the delivery of stimulation. For example, a user may define a routine that prevents or pauses stimulation, such as preventing or pausing stimulation when the patient is lying down (for example, if the patient finds such stimulation to hinder relaxation or sleeping) or driving or performing other activities. In at least some embodiments, the stimulation program can be a program that halts or starts a programmed stimulation or halts or starts a schedule of programmed stimulations. In at least some embodiments, the stimulation program can be a program that prevents changes in one or more of the stimulation parameters that could be disturbing to the patient (for example, changing stimulation amplitude while driving or resting.)

In step 306, the user selects or defines at least one activation criterion. The activation (or trigger) criterion that is selected or defined is based on information from a source external to the pulse generator that produces the stimulation according to the stimulation program. Meeting the activation criterion initiates the routine. Examples of activation criterion can include, but are not limited to, motion detection (for example, using a sensor), standing detection, lying detection, sleep detection, exercise detection, location detection (for example, at home, at the office, at the gym, in the car, or the like), a sensor measurement at a threshold level (for example, heart rate, pulse rate, oxygen level, glucose level, or the like at a threshold level or above/below), weather information (for example, temperature, pressure, humidity, at or above/below a threshold or the presence of precipitation), questionnaire responses, voice responses or commands, or the like or any combination thereof. In at least some embodiments, the activation criterion can include circumstances, such as sleeping or driving, during which the patient cannot provide manual input or would have difficulty providing manual input.

In at least some embodiments, the user can also specify at least one termination condition to terminate the routine. The termination condition can be, for example, a period of time or a total amount of stimulation delivered. The termination condition can be, for example, based on information from a source external to the pulse generator. Examples of termination conditions can include, but are not limited to, a stimulation time limit, a target number for delivery of stimulation pulses, location, cessation of activity (e.g., exercise, motion, standing, lying, or sleeping), or the like or any combination thereof.

In at least some embodiments, the user may specify multiple activation criteria or multiple termination conditions or any combination thereof. In at least some embodiments, the activation criteria/termination conditions can be associated with the same or different stimulation programs. In at least some embodiments, the routines can be programmed as if-then statements or loops or for-while loops or any other suitable structure.

In step 308, the user selects or defines a source to provide the information for the activation criterion or termination condition. Such a source can be, for example, a sensor, a website, a service, a RC 16, CP 18, or any other suitable device 21 (for example, a smartphone, smartwatch, tablet, laptop computer, desktop computer, personal data assistant, or the like), or the like or any combination thereof. Examples of sensors can include, but are not limited to, an accelerometer, a gyroscope, a global positioning system (GPS) locator, geofencing sensor(s), a pulse oximeter, or the like or any combination thereof. In at least some embodiments, the sensor can be located on the patient or can be included in a RC 16, CP 18, or any other suitable device 21 (for example, a smartphone, smartwatch, tablet, laptop computer, desktop computer, personal data assistant, or the like) or can be a device such as a voice assistant or web camera that is located near the patient. Examples of information from other sources include the weather, location of the patient, coupling to a WiFi network or Bluetooth^{™} device, coupling of the pulse generator to a charger, or the like. In at least some embodiments, the RC 16, CP 18, or other suitable device 21 can obtain the information from a website, service, an internal or external sensor, or any other suitable source.

In optional step 310, the pulse generator is programmed with the routine including the stimulation program, activation criterion, termination condition (if any), and source of the information. In other embodiments, the pulse generator may be programmed with the stimulation program, but another device, such as a RC 16, CP 18, or any other suitable device 21 (for example, a smartphone, smartwatch, tablet, laptop computer, desktop computer, personal data assistant, or the like), will direct the pulse generator to initiate the routine and, therefore, the pulse generator is not necessarily programmed with the activation criterion, termination condition, or source of information. In yet other embodiments, the RC 16, CP 18, or any other suitable device 21 (for example, a smartphone, smartwatch, tablet, laptop computer, desktop computer, personal data assistant, or the like) is programmed with the routine and communicates with the pulse generator to implement the routine on the pulse generator or to direct operation of the pulse generator according to the routine.

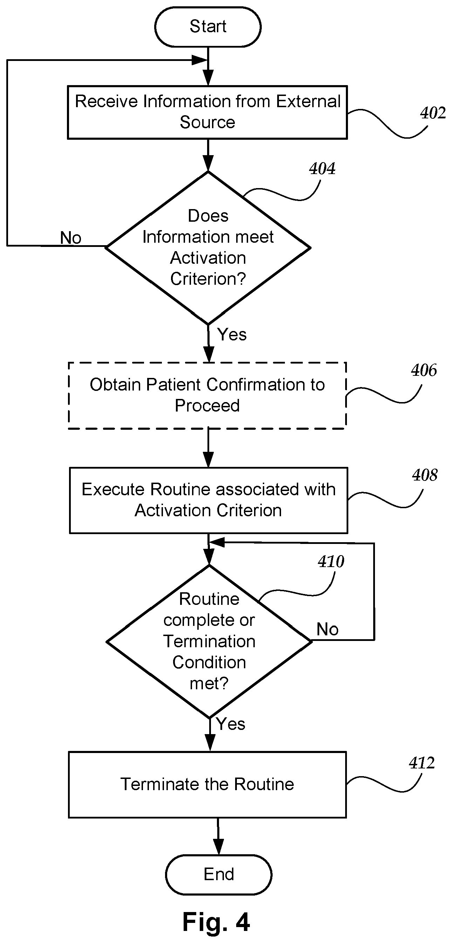

Figure 4 illustrates one embodiment of a method of performing a routine using a pulse generator. In step 402, a routine-containing device (for example, the pulse generator or a RC 16, CP 18, or any other suitable device 21) receives information from a source of information external to the pulse generator. The source can be, for example, a sensor, a website, a service, a RC 16, CP 18, or any other suitable device 21 (for example, a smartphone, smartwatch, tablet, laptop computer, desktop computer, personal data assistant, or the like), or the like or any combination thereof. In at least some embodiments, the routine-containing device communicates with the source of the information (for example, to a sensor or to a website) to ascertain when the activation criterion has been met or when a termination condition occurs. In other embodiments, the routine-containing device may communicate with a RC 16, CP 18, or any other suitable device 21 (for example, a smartphone, smartwatch, tablet, laptop computer, desktop computer, personal data assistant, or the like) to obtain the information.

In step 404, the routine-containing device determines if the information meets the activation criterion. If not, then the process returns to step 402. If yes, then in the process continues.

In optional step 406, the patient or other user is requested to confirm whether to proceed with the routine. For example, a pop-up message may be displayed on the RC 16, CP 18, or other device 21 (such as a smartphone or smartwatch) asking the patient or other user to confirm that the routine should proceed. In at least some embodiments, if the patient or user does not confirm then the routine does not proceed and the process ends.

In step 408, the routine is executed. In at least some embodiments, the routine-containing device is the pulse generator which implements the routine. In at least some embodiments, the routine-containing device is a RC 16, CP 18, or any other suitable device 21 which can direct the pulse generator to implement the routine or can direct operation of the pulse generator to implement the routine.

In step 410, the routine-containing device determines whether the routine is complete or a termination condition has been met. If not, the process continues. If yes, then the routine is terminated in step 412. The process can be repeated multiple times during operation of the pulse generator.

In at least some embodiments, steps 402 to 412 are executed by a RC 16, CP 18, or any other suitable device 21 (for example, a smartphone, smartwatch, tablet, laptop computer, desktop computer, personal data assistant, or the like). In step 408, the RC 16, CP 18, or any other suitable device 21 communicates with the pulse generator to execute the stimulation program of the routine and in step 412 the RC 16, CP 18, or any other suitable device 21 communicates with the pulse generator to terminate the stimulation program.

It will be understood that each block of the flowchart illustration, and combinations of blocks in the flowchart illustration and methods disclosed herein, can be implemented by computer program instructions. These program instructions may be provided to a processor to produce a machine or engine, such that the instructions, which execute on the processor, create means for implementing the actions specified in the flowchart block or blocks or engine disclosed herein. The computer program instructions may be executed by a processor to cause a series of operational steps to be performed by the processor to produce a computer implemented process. The computer program instructions may also cause at least some of the operational steps to be performed in parallel. Moreover, some of the steps may also be performed across more than one processor, such as might arise in a multi-processor computing device. In addition, one or more processes may also be performed concurrently with other processes, or even in a different sequence than illustrated without departing from the scope or spirit of the invention.

The computer program instructions can be stored on any suitable computer-readable medium including, but not limited to, RAM, ROM, EEPROM, flash memory or other memory technology, CD-ROM, digital versatile disks ("DVD") or other optical storage, magnetic cassettes, magnetic tape, magnetic disk storage or other magnetic storage devices, or any other medium which can be used to store the desired information and which can be accessed by a computing device. The computer program instructions can be stored locally or nonlocally (for example, in the Cloud).

The invention is defined in the appended claims. Aspects, embodiments and examples disclosed herein which do not fall within the scope of the appended claims do not form part of the invention and are merely provided for illustrative purposes. Furthermore, the methods presented in the present description are not claimed as such but are useful for understanding of the claimed subject matter, in particular since the methods can be performed by the claimed subject-matter.

## Claims

1. A stimulation system, comprising:
a device programmed with a plurality of user-specified routines for operation of a pulse generator (14, 20), wherein the user-specified routines are activatable in response to information received from a source external to the pulse generator, wherein the device comprises
a memory (205, 208) upon which the user-specified routines are stored,
a communication unit (209, 218) configured to receive or send signals, and
at least one processor (204, 207) coupled to the memory and the communication unit and configured to perform actions, comprising
receiving, using the communication unit, information provided from the source (220) external to the pulse generator and external to the patient, wherein the information is selected from sensor information, patient location, weather information, or attachment to a charger,
when the information meets an activation criterion for a one of the user-specified routines, activating the user-specified routine to deliver or halt delivery of stimulation to the patient according to the user-specified routine, and
upon completion of the user-specified routine or meeting a termination condition, halting the user-specified routine.

2. The stimulation system of claim 1, wherein the device is the pulse generator which is an implantable pulse generator (14) or an external trial stimulator (20).

3. The stimulation system of any one of claims 1 or 2, further comprising a sensor comprising an antenna for communicating with the device and providing the sensor information, wherein the sensor is configured to provide a sensor measurement indicating at least one of the following: movement by the patient, that the patient is asleep, that the patient is driving, that the patient is exercising, pain, disease/disorder state, or disease/disorder progression.

4. The stimulation system of any one of claims 1 to 3, wherein the user-specified routines are activatable in response to information received from the source that is external to the patient.

5. The stimulation system of any one of claims 1 to 4, wherein the user-specified routines are activatable in response to information received from the source that is external to the device.

6. The stimulation system of any one of claims 1 to 5, further comprising a remote control (16), clinician programmer (18), or mobile device (21) comprising a communication unit for communicating with the device and providing the information.

7. The stimulation system of any one of claims 1 to 6, further comprising a charger (22), wherein the source of the information is the charger.

8. The stimulation system of any one of claims 1 to 7, wherein the device is a remote control, clinician programmer, smartphone, or smartwatch configured for communicating with the pulse generator using the communication unit, wherein the actions further comprise, when the information meets the activation criterion for the one of the user-specified routines and prior to activating the one of the user-specified routines, presenting a message on the remote control, clinician programmer, smartphone, or smartwatch requesting confirmation of the activation of the one of the user-specified routines by the patient.

9. A stimulation system (10, 200), comprising:
a display (213);
at least one user input device (211); and
at least one processor (207) coupled to the display and user input device, the at least one processor configured to perform actions, comprising:
providing a user interface on the display;
receiving, via the at least one user input device, a user-selection of a stimulation program for a user-specified routine for stimulation of a patient using a pulse generator;
receiving, via the at least one user input device, a user-selection of an activation criterion for the user-specified routine; and
receiving, via the at least one user input device, a user-selection of a source external to the pulse generator to provide the activation criterion, wherein the activation criterion is selected from motion detection, standing detection, lying detection, sleep detection, exercise detection, location detection, a sensor measurement at a threshold level, or weather information.

10. The stimulation system of claim 9, wherein the actions further comprise initiating a signal to program the pulse generator to include the user-selection of the stimulation program, activation criterion, and source for the user-specified routine.

11. The stimulation system of any one of claims 9 or 10, wherein the actions further comprising receiving, via the at least one user input device, a termination condition.

12. The stimulation system of any one of claims 9 to 11, wherein receiving the user-selection of the activation criterion and receiving the user-selection of the source comprise receiving, via the at least one user input device, a user-selection of a predefined routine from a database.

13. The stimulation system of any one of claims 9 to 12, wherein the actions further comprise presenting a suggested routine to the user on the user interface based on observations of previous patient behavior or previous user or patient selections of stimulation programs.

14. The stimulation system of any one of claims 9 to 13, wherein the actions further comprise receiving, via the at least one user input device, a user-selection of an option to require confirmation of an activation of the user-specified routine by the user when activating the user-specified routine.

15. The stimulation system of any one of claims 9 to 14, further comprising a remote control (16), clinician programmer (18), or mobile device (21), wherein the display, the user input device, and the at least one processor are part of the remote control, clinician programmer, or mobile device.

## Patentansprüche

1. Stimulationssystem, aufweisend:
eine Vorrichtung, die mit einer Vielzahl von benutzerspezifischen Routinen zum Betreiben eines Pulsgenerators (14, 20) programmiert ist, wobei die benutzerspezifischen Routinen in Antwort auf Information aktivierbar sind, die von einer Quelle außerhalb des Pulsgenerators empfangen wird, wobei die Vorrichtung aufweist:
einen Speicher (205, 208), auf dem die benutzerspezifischen Routinen gespeichert sind, eine Kommunikationseinheit (209, 218), die dafür konfiguriert ist, Signale zu empfangen oder zu übertragen, und
mindestens einen Prozessor (204, 207), der mit dem Speicher und der Kommunikationseinheit verbunden ist und dafür konfiguriert ist, Aktionen auszuführen, aufweisend:
Empfangen von Information, die von der Quelle (220) außerhalb des Pulsgenerators und außerhalb des Patienten bereitgestellt wird, unter Verwendung der Kommunikationseinheit, wobei die Information aus Sensorinformation, Patientenstandort, Wetterinformation oder Anschluss an ein Ladegerät ausgewählt wird;
wenn die Information ein Aktivierungskriterium für eine der benutzerspezifischen Routinen erfüllt, Aktivieren der benutzerspezifischen Routine, um eine Stimulation für den Patienten gemäß der benutzerspezifischen Routine bereitzustellen oder anzuhalten; und
nach Abschluss der benutzerspezifischen Routine oder Erfüllen einer Beendigungsbedingung, Anhalten der benutzerspezifischen Routine.

2. Stimulationssystem nach Anspruch 1, wobei die Vorrichtung der Pulsgenerator ist, der ein implantierbarer Pulsgenerator (14) oder ein externer Teststimulator (20) ist.

3. Stimulationssystem nach Anspruch 1 oder 2, ferner aufweisend einen Sensor, der eine Antenne zum Kommunizieren mit der Vorrichtung und zum Bereitstellen der Sensorinformation aufweist, wobei der Sensor dafür konfiguriert ist, eine Sensormessung bereitzustellen, die mindestens eines der folgenden Merkmale anzeigt: Bewegung des Patienten, dass der Patient schläft, dass der Patient fährt, dass der Patient trainiert, Schmerzen, Krankheits-/Störungszustand oder Krankheits-/Störungsprogression.

4. Stimulationssystem nach einem der Ansprüche 1 bis 3, wobei die benutzerspezifischen Routinen in Antwort auf Information aktivierbar sind, die von der Quelle empfangen werden, die sich außerhalb des Patienten befindet.

5. Stimulationssystem nach einem der Ansprüche 1 bis 4, wobei die benutzerspezifischen Routinen in Antwort auf Information aktivierbar sind, die von der Quelle empfangen wird, die sich außerhalb der Vorrichtung befindet.

6. Stimulationssystem nach einem der Ansprüche 1 bis 5, ferner aufweisend eine Fernbedienung (16), ein Programmiergerät für Ärzte (18) oder ein mobiles Gerät (21), das eine Kommunikationseinheit zum Kommunizieren mit der Vorrichtung und zum Bereitstellen der Information aufweist.

7. Stimulationssystem nach einem der Ansprüche 1 bis 6, ferner aufweisend ein Ladegerät (22), wobei die Quelle der Information das Ladegerät ist.

8. Stimulationssystem nach einem der Ansprüche 1 bis 7, wobei die Vorrichtung eine Fernbedienung, ein Programmiergerät für Ärzte, ein Smartphone oder eine Smartwatch ist, die für eine Kommunikation mit dem Pulsgenerator unter Verwendung der Kommunikationseinheit konfiguriert sind, wobei die Aktionen ferner aufweisen, wenn die Information das Aktivierungskriterium für die eine der benutzerspezifischen Routinen erfüllt und vor dem Aktivieren der einen der benutzerspezifischen Routinen, Anzeigen einer Meldung auf der Fernbedienung, dem Programmiergerät für Ärzte, dem Smartphone oder der Smartwatch, die eine Bestätigung der Aktivierung der einen der benutzerspezifischen Routinen durch den Patienten anfordert.

9. Stimulationssystem (10, 200), aufweisend:
eine Anzeige (213);
mindestens ein Benutzereingabegerät (211); und
mindestens einen Prozessor (207), der mit der Anzeige und dem Benutzereingabegerät gekoppelt ist, wobei der mindestens eine Prozessor dafür konfiguriert ist, Aktionen auszuführen, die aufweisen:
Bereitstellen einer Benutzerschnittstelle auf der Anzeige;
Empfangen einer Benutzerauswahl eines Stimulationsprogramms für eine benutzerspezifische Routine zum Stimulieren eines Patienten unter Verwendung eines Pulsgenerators über das mindestens eine Benutzereingabegerät;
Empfangen einer Benutzerauswahl eines Aktivierungskriteriums für die benutzerspezifische Routine über das mindestens eine Benutzereingabegerät; und
Empfangen einer Benutzerauswahl einer Quelle außerhalb des Pulsgenerators zum Bereitstellen des Aktivierungskriteriums über das mindestens eine Benutzereingabegerät, wobei das Aktivierungskriterium aus Bewegungserfassung, Steherfassung, Liegeerfassung, Schlaferfassung, Trainingserfassung, Standorterfassung, einer Sensormessung bei einem Schwellenwert oder Wetterinformation ausgewählt wird.

10. Stimulationssystem nach Anspruch 9, wobei die Aktionen ferner aufweisen:
Auslösen eines Signals zum Programmieren des Pulsgenerators, um die Benutzerauswahl des Stimulationsprogramms, das Aktivierungskriterium und die Quelle für die benutzerspezifische Routine einzubeziehen.

11. Stimulationssystem nach Anspruch 9 oder 10, wobei die Aktionen ferner das Empfangen einer Beendigungsbedingung über das mindestens eine Benutzereingabegerät aufweisen.

12. Stimulationssystem nach einem der Ansprüche 9 bis 11, wobei das Empfangen der Benutzerauswahl des Aktivierungskriteriums und das Empfangen der Benutzerauswahl der Quelle das Empfangen einer Benutzerauswahl einer vordefinierten Routine von einer Datenbank über das mindestens eine Benutzereingabegerät aufweisen.

13. Stimulationssystem nach einem der Ansprüche 9 bis 12, wobei die Aktionen ferner das Präsentieren einer vorgeschlagenen Routine für den Benutzer auf der Benutzerschnittstelle basierend auf Beobachtungen von früherem Patientenverhalten oder früheren Benutzer- oder Patientenauswahlen von Stimulationsprogrammen aufweisen.

14. Stimulationssystem nach einem der Ansprüche 9 bis 13, wobei die Aktionen ferner das Empfangen einer Benutzerauswahl einer Option zum Anfordern einer Bestätigung einer Aktivierung der benutzerspezifischen Routine durch den Benutzer bei Aktivierung der benutzerspezifischen Routine über das mindestens eine Benutzereingabegerät aufweisen.

15. Stimulationssystem nach einem der Ansprüche 9 bis 14, ferner aufweisend eine Fernbedienung (16), ein Programmiergerät für Ärzte (18) oder ein mobiles Gerät (21), wobei die Anzeige, das Benutzereingabegerät und der mindestens eine Prozessor Teil der Fernbedienung, des Programmiergeräts für Ärzte oder des mobilen Geräts sind.

## Revendications

1. Système de stimulation, comprenant :
un dispositif programmé avec une pluralité de routines spécifiées par l'utilisateur pour le fonctionnement d'un générateur d'impulsions (14, 20), dans lequel les routines spécifiées par l'utilisateur peuvent être activées en réponse à des informations reçues en provenance d'une source externe au générateur d'impulsions, dans lequel le dispositif comprend
une mémoire (205, 208) sur laquelle sont stockées les routines spécifiées par l'utilisateur,
une unité de communication (209, 218) configurée pour recevoir ou envoyer des signaux, et
au moins un processeur (204, 207) couplé à la mémoire et à l'unité de communication et configuré pour effectuer des actions, comprenant le fait de
recevoir, à l'aide de l'unité de communication, des informations fournies par la source (220) externe au générateur d'impulsions et externe au patient, dans lesquelles les informations sont sélectionnées parmi des informations de capteur, un emplacement de patient, des informations météorologiques, ou une connexion pour un chargeur,
lorsque les informations satisfont un critère d'activation pour l'une des routines spécifiées par l'utilisateur, activer la routine spécifiée par l'utilisateur pour délivrer la stimulation au patient, ou arrêter la délivrance de la stimulation au patient, selon la routine spécifiée par l'utilisateur, et
à l'achèvement de la routine spécifiée par l'utilisateur, ou lorsqu'une condition de fin est satisfaite, arrêter la routine spécifiée par l'utilisateur.

2. Système de stimulation selon la revendication 1, dans lequel le dispositif est le générateur d'impulsions qui est un générateur d'impulsions implantable (14) ou un stimulateur d'essai externe (20).

3. Système de stimulation selon l'une quelconque des revendications 1 ou 2, comprenant en outre un capteur comprenant une antenne pour communiquer avec le dispositif et fournir les informations de capteur, dans lequel le capteur est configuré pour fournir une mesure de capteur indiquant au moins l'un des éléments suivants : un mouvement du patient, le fait que le patient est endormi, le fait que le patient conduit, le fait que le patient fait de l'exercice, de la douleur, un état de maladie/trouble ou une progression de maladie/trouble.

4. Système de stimulation selon l'une quelconque des revendications 1 à 3, dans lequel les routines spécifiées par l'utilisateur peuvent être activées en réponse à des informations reçues en provenance de la source qui est externe au patient.

5. Système de stimulation selon l'une quelconque des revendications 1 à 4, dans lequel les routines spécifiées par l'utilisateur peuvent être activées en réponse à des informations reçues en provenance de la source qui est externe au dispositif.

6. Système de stimulation selon l'une quelconque des revendications 1 à 5, comprenant en outre une commande à distance (16), un programmateur clinique (18), ou un dispositif mobile (21) comprenant une unité de communication pour communiquer avec le dispositif et fournir les informations.

7. Système de stimulation selon l'une quelconque des revendications 1 à 6, comprenant en outre un chargeur (22), dans lequel la source des informations est le chargeur.

8. Système de stimulation selon l'une quelconque des revendications 1 à 7, dans lequel le dispositif est une commande à distance, un programmateur clinique, un téléphone intelligent ou une montre connectée configuré(e) pour communiquer avec le générateur d'impulsions à l'aide de l'unité de communication, dans lequel les actions comprennent en outre le fait de, lorsque les informations satisfont le critère d'activation de l'une des routines spécifiées par l'utilisateur, et avant d'activer ladite une des routines spécifiées par l'utilisateur, présenter un message sur la commande à distance, le programmateur clinique, le téléphone intelligent ou la montre connectée, demandant la confirmation, par le patient, de l'activation de ladite une des routines spécifiées par l'utilisateur.

9. Système de stimulation (10, 200), comprenant :
un écran (213) ;
au moins un dispositif d'entrée utilisateur (211) ; et
au moins un processeur (207) couplé à l'écran et au dispositif d'entrée utilisateur, l'au moins un processeur étant configuré pour effectuer des actions, comprenant le fait de :
fournir une interface utilisateur sur l'écran ;
recevoir, par l'intermédiaire de l'au moins un dispositif d'entrée utilisateur, une sélection, par l'utilisateur, d'un programme de stimulation, pour une routine spécifiée par l'utilisateur en vue de la stimulation d'un patient à l'aide d'un générateur d'impulsions ;
recevoir, par l'intermédiaire du dispositif d'entrée utilisateur, une sélection, par l'utilisateur, d'un critère d'activation pour la routine spécifiée par l'utilisateur ; et
recevoir, par l'intermédiaire du dispositif d'entrée utilisateur, une sélection, par l'utilisateur, d'une source externe au générateur d'impulsions pour fournir le critère d'activation, dans lequel le critère d'activation est sélectionné parmi une détection de mouvement, une détection de position debout, une détection de position couchée, une détection de sommeil, une détection d'exercice, une détection d'emplacement, une mesure de capteur à un niveau de seuil, ou des informations météorologiques.

10. Système de stimulation selon la revendication 9, dans lequel les actions comprennent en outre le fait d'initier un signal pour programmer le générateur d'impulsions afin d'inclure la sélection, par l'utilisateur, du programme de stimulation, du critère d'activation, et de la source pour la routine spécifiée par l'utilisateur.

11. Système de stimulation selon l'une quelconque des revendications 9 et 10, dans lequel les actions comprennent en outre le fait de recevoir, par l'intermédiaire de l'au moins un dispositif d'entrée utilisateur, une condition de fin.

12. Système de stimulation selon l'une quelconque des revendications 9 à 11, dans lequel la réception de la sélection, par l'utilisateur, du critère d'activation, et la réception de la sélection, par l'utilisateur, de la source, comprennent le fait de recevoir, par l'intermédiaire de l'au moins un dispositif d'entrée utilisateur, une sélection, par l'utilisateur, d'une routine prédéfinie, en provenance d'une base de données.

13. Système de stimulation selon l'une quelconque des revendications 9 à 12, dans lequel les actions comprennent en outre le fait de présenter une routine suggérée, à l'utilisateur, sur l'interface utilisateur, sur la base d'observations d'un comportement de patient antérieur ou de sélections antérieures, par un utilisateur ou un patient, de programmes de stimulation.

14. Système de stimulation selon l'une quelconque des revendications 9 à 13, dans lequel les actions comprennent en outre le fait de recevoir, par l'intermédiaire de l'au moins un dispositif d'entrée utilisateur, une sélection, par l'utilisateur, d'une option pour exiger la confirmation, par l'utilisateur, d'une activation de la routine spécifiée par l'utilisateur, lors de l'activation de la routine spécifiée par l'utilisateur.

15. Système de stimulation selon l'une quelconque des revendications 9 à 14, comprenant en outre une commande à distance (16), un programmateur clinique (18), ou un dispositif mobile (21), dans lequel l'écran, le dispositif d'entrée utilisateur et l'au moins un processeur font partie de la commande à distance, du programmateur clinique, ou du dispositif mobile.
